# EUROPEAN PATENT SPECIFICATION

(11) **EP 3 265 008 B1**
(45) Date of publication and mention of the grant of the patent: **05.06.2024**
(21) Application number: 16709863.1
(22) Date of filing: 23.02.2016
(51) Int. Cl.: A61B 3/13, A61B 34/00, G06F 3/01, A61B 90/20, G06F 1/16, G06F 3/0482, A61B 34/20, A61B 17/00, A61B 90/00

(54) **SURGICAL TOOL TRACKING TO CONTROL SURGICAL SYSTEM**
VERFOLGUNG EINES CHIRURGISCHEN INSTRUMENTS ZUR STEUERUNG EINES CHIRURGISCHEN SYSTEMS
SUIVI D'OUTIL CHIRURGICAL POUR COMMANDER UN SYSTÈME CHIRURGICAL

(30) Priority: 14.05.2015 US 201514712186
(43) Date of publication of application: 10.01.2018
(73) Proprietor: Alcon Inc., 1701 Fribourg (CH)
(72) Inventor: REN, Hugang, Lake Forest, California 92630 (US); YU, Lingfeng, Rancho Santa Margarita, California 92688 (US)
(74) Representative: Murgitroyd & Company
(86) International application number: PCT/US2016/019146
(87) International publication number: WO 2016/182611

(56) References cited:
- WO-A1-2011/085815
- WO-A1-2014/170236
- WO-A2-2016/109280
- DE-A1-102004 049 258
- US-A1- 2012 071 891
- US-A1- 2014 005 484
- US-A1- 2014 221 822

## Description

### BACKGROUND OF THE INVENTION

The present invention relates to surgical systems and more particularly to a surgical system using a surgical control tool as a control input.

Accurate surgical settings are critical to the success of a surgery. Therefore, when surgical conditions change during surgery, the ability to adjust the surgical settings is highly desired, especially for delicate ophthalmic surgeries. Modern surgical consoles are designed to have different operation modes and settings tailored to each specific task. For instance, a vitreoretinal surgical console may be equipped with three different modes for a vitrectomy procedure, including CORE, SHAVE and 50/50. When a vitretomy procedure starts, the console is configured in CORE mode so that most of the vitreous cortex can be removed efficiently. After that, the console needs to be manually configured into SHAVE mode in order to safely shave the vitreous base at the peripheral. Moreover, even within the same surgical mode, the surgeon may want to change some of the settings based on different surgical conditions. For example, if a retinal hemorrhage occurs during vitrectomy, the surgeon will immediately increase the intraocular pressure (IOP) to try to stop the bleeding.

In current ophthalmic surgical practice, control of surgical settings is performed either by an assistant through a touch screen several feet away from the surgeon or by the surgeon through a foot pedal. If it is performed by an assistant, the surgeon will have to verbally communicate with the assistant first, and then wait until the assistant finishes the action assuming that the assistant will always understand the surgeon's request correctly. Also, it increases the manpower requirement for a given surgery. On the other hand, if it is performed by the surgeon through a foot pedal, it will not involve any of the complexities mentioned above. However, the foot pedal is a physical device which can only accommodate a limited number of control commands.

Therefore, there is a need for a surgical system empowering the surgeon with full control over the surgical settings without increasing the complexity of the current surgical consoles, potentially realizing assistant-free surgery. Reference is made to US 2012/071891 and US 2014/005484 which relate to different prior art tracking arrangements. WO 2016/109280 (a document published after the priority date of the present application) provide some tracking for visualisation application including analysis of depth and magnification of the region of interest respectively.

US 2014/0221822A1 discloses a system for tracking a depth of a surgical instrument in an optical coherence tomography (OCT) guided surgical procedure. An OCT device is configured to image a region of interest to provide OCT data. A scan processor is configured to determine a relative position of the instrument and a target within the region of interest from at least the OCT data, where the instrument is one of in front of the target, within the target, or below the target. A feedback element is configured to communicate the relative position of the instrument and the target to a user in a human comprehensible form. This document does not disclose that the control unit is configured to identify a control action by associating the control input unit and the temporal spatial information of the vitreoretinal surgical tool to identify whether the temporal spatial information is associated with a motion pattern of a control command of the control input unit.

### SUMMARY OF THE INVENTION

The invention is set out in the appended set of claims.

Accordingly, there is provided a surgical system in accordance with claim 1. The dependent claims provide further optional features.

The specification also provides description of arrangements not claimed in the present claims but provided as background and to assist in understanding the invention, these arrangements of the specification include the methods of controlling and operating the system of the claims.

The present invention discloses a surgical system as claimed in claim 1 which comprises a heads-up display, a surgical microscope, a control unit, a surgical tool, a tracking unit for tracking a motion of the surgical tool and a processing unit processing the motion of the surgical tool to obtain a temporal spatial information of the surgical tool. The control unit further comprises a control input unit comprising a number of control commands. The control unit identifies a control action by associating the control input unit and the temporal spatial information of the surgical tool and applies a corresponding control command to the surgical system.

The tracking unit may be a software based tool tracking unit. It comprises an imaging unit, capturing at least one image of the surgical tool and a surgical site. The imaging unit may be optical camera, interferometer, infrared camera, etc. The tracking unit may be a hardware based tool tracking unit as well. For instance, the tracking unit may comprise one or more tracking sensors such as gyroscope, magnetic sensor, optical sensor, accelerometer, etc.

The control input unit comprises a number of control commands. Each of the control commands can be associated with or encoded into a motion pattern/gesture respectively. Each of the control commands may also be designed as a button/icon respectively. The button/icon can display and/or update parameters of various surgical settings.

The temporal spatial information of the surgical tool contains such information of the surgical tool as motion profile, motion pattern/gesture, location, rotation direction, tool angle, tip proximity from the surgical site, speed, orientation, length, number, etc. The temporal spatial information of the surgical tool may contain information of a distal end of the surgical tool, any part of the surgical tool or the whole surgical tool.

The present disclosure further describes several examples of the invention. In one example the present invention, the control input unit could further comprise a virtual Graphic User Interface (GUI) configured in the surgical microscope. The virtual GUI can be displayed through a heads-up display and each of the control commands is designed as a button or icon inside the virtual GUI. The control commands could be designed depending on different applications. The virtual GUI could be displayed in a virtual plane a distance from a surgical site or in a periphery of the surgical site while the control commands could be designed in a center of the virtual GUI or in a periphery of the virtual GUI.

In another example of the present invention, the surgical system comprises two tracking units (e.g., imagining units) such that a stereovision of the surgical site can be achieved. 3D tool tracking can be performed to extract 3D motion. In such a surgical system, the temporal spatial information of the surgical tool can contain 3D information.

In another example of the present invention, the tracking unit of the surgical system comprises one or more tracking sensors connected to the surgical tool. The tracking unit can further generate a 3D motion. In such a surgical system, the temporal spatial information of the surgical tool can contain 3D information. One or more tracking sensors may be coupled to the surgical.

In another example, the system comprises an output unit for interacting with a user. The output unit may be a speaker (and a microphone) such that the surgical system can warn the user to the surgical tool away from a tissue or inform the user that the control action has been identified before the corresponding control command is applied. The output unit also may be a heads-up display displaying a virtual GUI such that the virtual GUI can update/inform the user of a status of the surgical system and/or enable the user to confirm the corresponding control command.

### BRIEF DESCRIPTION OF THE FIGURES

The accompanying drawings, which are incorporated in and constitute a part of this specification, illustrate several embodiments of the invention and together with the description, serve to explain the principles of the invention.
Figure 1 is a schematic representation of one embodiment of an ophthalmic surgical console.
Figure 2 is a representation of one embodiment of a surgical system.
Figures 3a-3h are schematic diagrams of exemplary motion patterns/gestures as control commands.
Figure 4 is a representation of one embodiment of a surgical system with 3D tracking.
Figure 5 is a representation of one embodiment of a surgical system with tracking sensor.
Figures 6a-6c are schematic diagrams of view of surgical site without and with a virtual Graphic User Interface (GUI) and a surgical tool.
Figures 7a-7c are schematic diagrams of view of user with different user focuses.
Figure 8 is a representation of one arrangement of a surgical system control method according to the specification and outside the scope of the present claims.
Figures 9a and 9b are representations of two control modes according to the specification.
Figure 10 is a representation of another arrangement of a surgical system control method outside the scope of the present claims with a virtual GUI.
Figures 11a and 11b are representations of two control modes with the virtual GUI.

### DETAILED DESCRIPTION

Reference is now made in detail to the exemplary embodiments of the invention, examples of which are illustrated in the accompanying drawings. Wherever possible, the same reference numbers are used throughout the drawings to refer to the same or like parts.

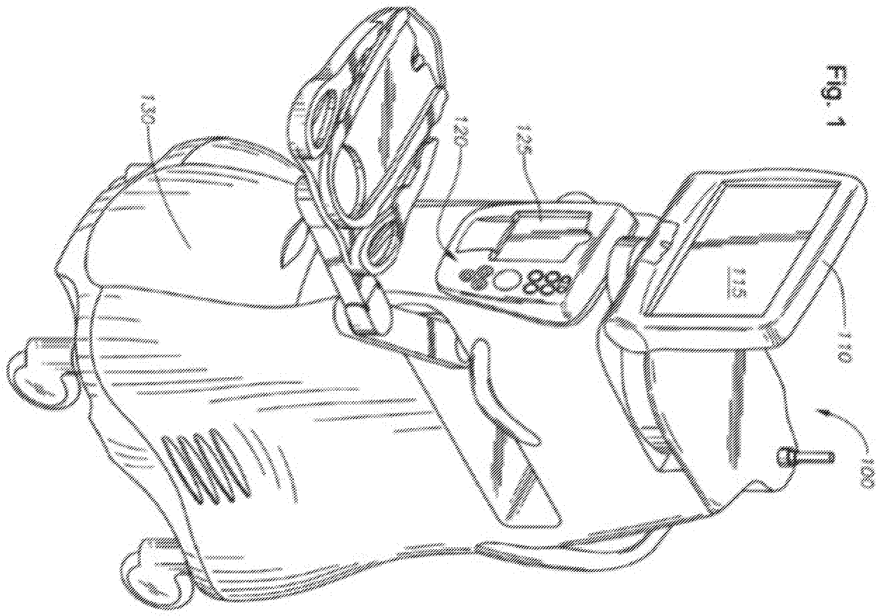

Figure 1 is a diagrammatic representation of one embodiment of an ophthalmic surgical console 100. Surgical console 100 can include a swivel monitor 110 that has touch screen 115. Swivel monitor 110 can be positioned in a variety of orientations for whomever needs to see touch screen 115. Swivel monitor 110 can swing from side to side, as well as rotate and title. Touch screen 115 provides a Graphic User Interface (GUI) that allows a user to interact with console 100.

Surgical console 100 also includes a connection panel 120 used to connect various tools and consumables to surgical console 100. Connection panel 120 can include, for example, a coagulation connector, balanced salt solution receiver, connectors for various hand pieces and a fluid management system (FMS) or cassette receiver 125. Surgical console 100 can also include a variety of user friendly features, such as a foot pedal control (e.g., stored behind panel 130) and other features. In operation, a cassette (not shown) can be placed in cassette receiver 125 and held in place with clamps to minimize movement during use.

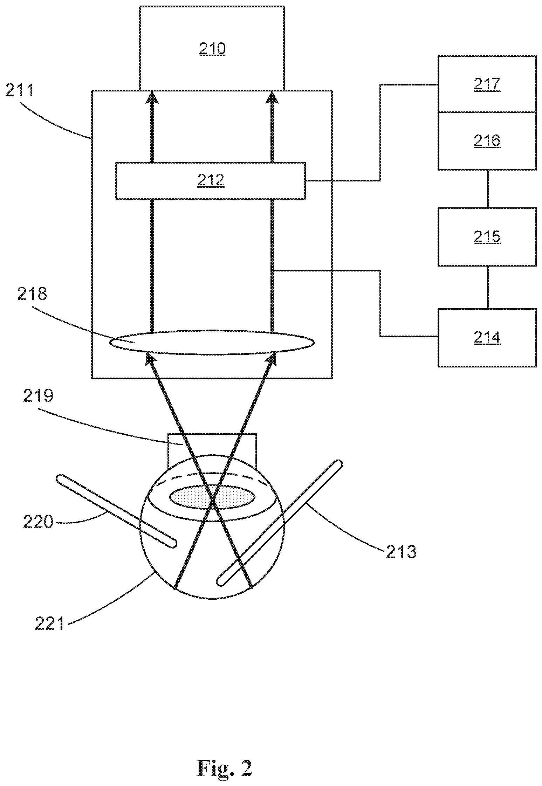

Figure 2 is a representation of one embodiment of a surgical system. Without loss of generality, hereinafter a vitreoretinal system has been selected as an example. Other surgical systems, such as cataract surgical systems may also employ the systems and methods described herein, the methods are outside the scope of the present claims.

The example of figure 2, one exemplary system used to enable surgical tool as a control input in vitreoreinal surgery is based on software tracking. The surgical system comprises an eyepiece 210, a microscope 211, a heads-up display 212 configured in the surgical microscope 211, a control unit 217, a surgical tool 213, an imaging unit 214 tracking a motion of the surgical tool 213 and capturing at least one image of the surgical tool 213 and a surgical site, and a processing unit 215 processing the motion of the surgical tool 213 to obtain a temporal spatial information of the surgical tool 213. The control unit 217 further comprises a control input unit 216 comprising a number of control commands associated with or encoded into various motion patterns/gestures, such that the control unit 217 identifies a control action by associating the control input unit 216 and the temporal spatial information of the surgical tool 213 and displays a corresponding control command through the heads-up display 212. The surgical tool 213 could be placed in anterior segment and/or posterior segment of an eye 221 during a surgery. It should be understood that the imaging unit can be designed to track the motion of the whole surgical tool 213, part of the surgical tool 213 or the distal tip of the surgical tool 213. An objective lens 218 can be configured in the microscope 211 such that the objective lens 218 could adjust a user's focus either on the surgical tool 213 or the surgical site. An illuminator 220 may be deployed in the eye 221 as a light source. Moreover, a surgical lens 219 may be coupled to the eye 221 in a direct or indirect means.

The surgical site can be seen through the eyepiece 210 with the microscope 211. During the surgery, the imaging unit 214 (e.g., a video camera) tracks the motion of the surgical tool 213 by capturing at least one image and/or a video of the surgical tool 213 and the surgical site. The processing unit 215 receives the images and/or the video and enhances and processes the image and/or the video to extract the motion of the surgical tool 213 so as to obtain temporal spatial information of the surgical tool 213. The control commands in the control input unit are associated with or encoded into various motion patterns/gestures. Thus, based on the identified motion pattern/gesture enclosed in the temporal spatial information, the control unit 215 associates the identified motion pattern/gesture with the associated or encoded control commands in the control input unit 216 with the control commands and ascertains whether the identified motion pattern/gesture is a control action. If it is a control action, the control unit 217 later extracts a corresponding control command related to the control action and then alternatively displays the corresponding control command through the heads-up display 212 for user's confirmation. Once the user confirms the control command, the corresponding control command is applied to the surgical system. Alternatively the updated system status could be displayed on the virtual GUI for user's information.

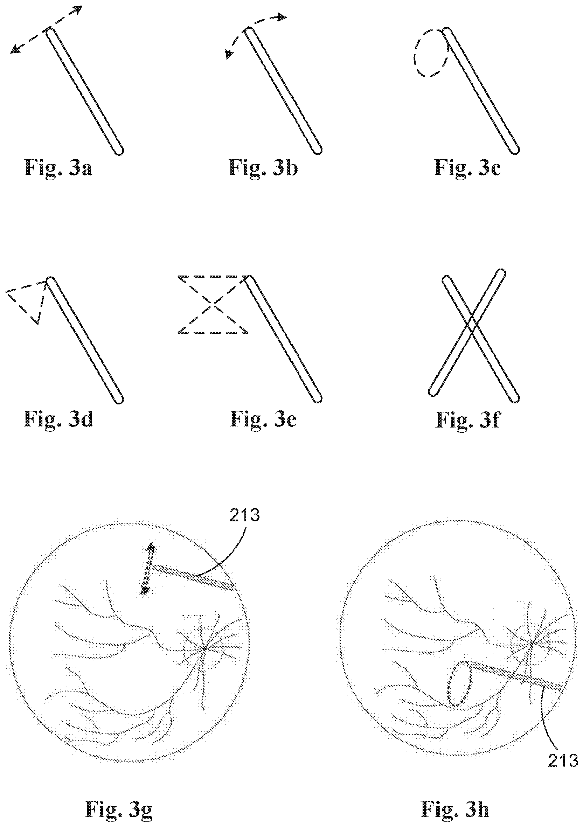

Figures 3a to 3h are schematic diagrams of exemplary motion patterns/gestures as control commands. Figures 3a to 3f show some of the exemplary motion patterns/gestures of the distal end of the surgical tool that can be control commands. Figure 3a shows a motion pattern of multiple linear translations of the surgical tool 213. The number of repeating lines, orientation, length, speed, etc. of the motion profiles can be used to encode control commands. Figure 3b shows a motion pattern of clock-wise and counter-clock wise rotations of the surgical tool 213. The direction, rotation speed can be used to encode control commands. For example, the clock-wise rotation may be associated with a command to increase intra-ocular pressure (IOP) while the counter-clock wise rotation may be associated with a command to decrease IOP. Figure 3c shows a motion pattern of a circular/elliptical shape. The direction, diameter, rotation speed may be used as motion control commands. Figure 3d shows a triangular shape motion pattern, which represents a group of motion patterns with polygonal shape. Figure 3e shows a figure-eight-shaped pattern representing any arbitrarily designed motion patterns that can be drawn continuously. Figure 3f shows a gesture created by two surgical tools such as the illuminator 220 and the surgical tool 213 crossing each other, which represents a group of many gestures that can be used to encode various control commands. These patterns/gestures are exemplary in nature. Motion patterns and gestures can also be combined to achieve more advanced surgical controls with one or multiple tools. Figure 3g illustrates the user's view including the surgical site, the surgical tool and the corresponding motion profile. Similar to figure 3g, figure 3h shows not only the motion patterns/gestures, but also the location of the motion pattern/gesture. In this example, the location of the motion patterns/gestures can be associated with a control command. In this manner both the motion itself and the location of the tool in the eye can be associated with a control command.

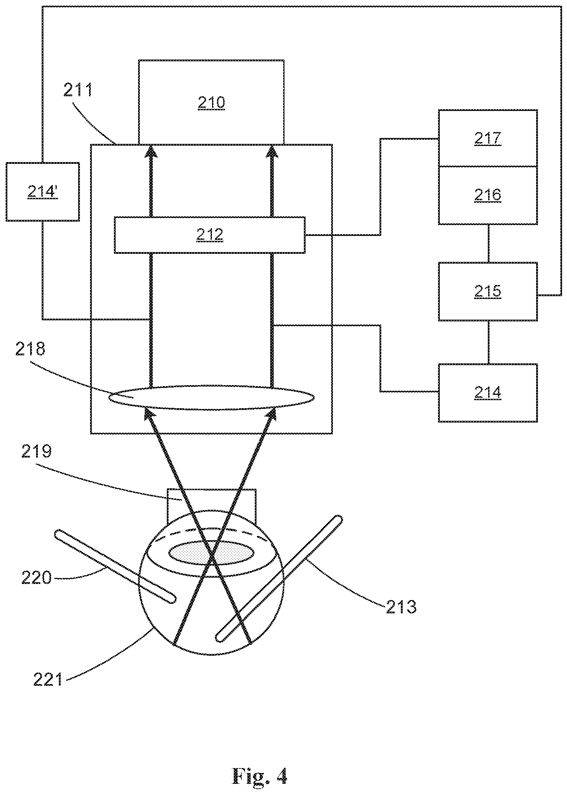

Figure 4 is a representation of another embodiment of a surgical system with 3D tracking. In the example of Figure 4, a second imaging unit 214' is employed to achieve stereovision of the motion of the surgical tool and the surgical site. 3D tool tracking can then be performed to extract 3D motion patterns/gestures, providing more control freedom to the user. In this example, the temporal spatial information of the surgical tool 213 contains 3D information. The control commands in the control input unit 216 could be correspondingly associated with or encoded into various 3D motion profiles such as 3D motion patterns/gestures. The use of 3D information expands the potential range of patterns/gestures that can be associated with control commands. In another example, 3D information can be combined with the location of the pattern/gesture and both can be associated with a control command. The location of the gesture may indicate a location at which a command is to be performed.

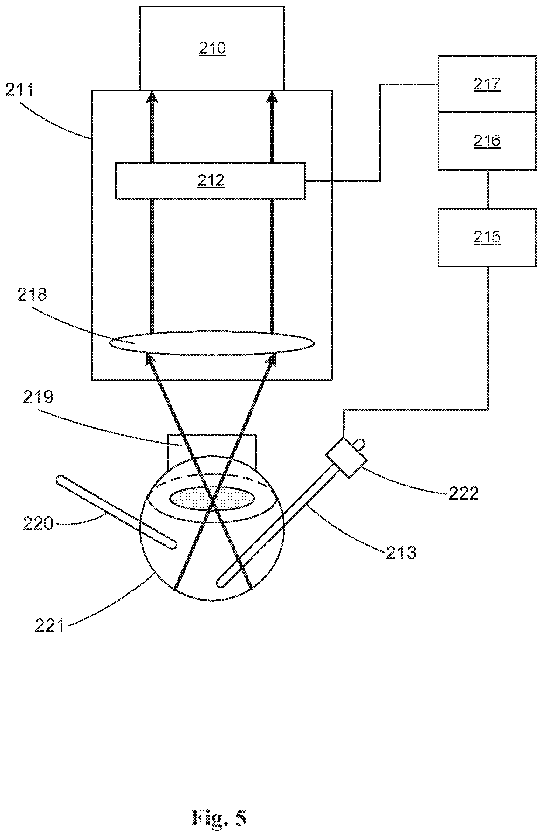

Figure 5 is a representation of one embodiment of a surgical system with a tracking sensor. In the example of Figure 5, the system is designed based on hardware tracking to enable the surgical tool as a control input unit for surgical system. One or multiple tracking sensors 222 (e.g., gyroscope, magnetic sensor, optical sensor, accelerometer, etc.) are coupled to the surgical tool. The readings from these tracking sensors can be used to extract the 2D and/or 3D motion patterns/gestures of the surgical tool. Corresponding control command can be associated with the 2D and/or 3D motion patterns/gestures of the surgical tool as previously described.

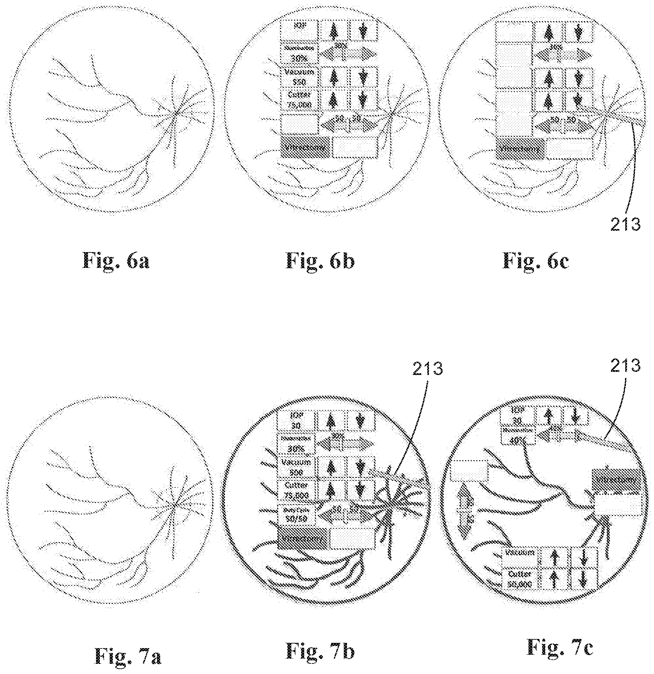

Figures 6a to 6c are schematic diagrams of a view of surgical site without and with a virtual GUI and a surgical tool. Figure 6a shows the image of the user's view of the surgical site without a virtual GUI and a surgical tool. Figure 6b shows the image of the user's view of the surgical site with a virtual GUI. Figure 6c shows the image of the user's view of the surgical site with a virtual GUI and a surgical tool. When the tool control is enabled, a virtual GUI is then displayed through the heads-up display to the user, as shown in figure 6b.

In this example, several commonly used settings for vitrectomy surgery are displayed. For instance, control commands/settings such as IOP, illumination, vacuum, cutter speed, duty cycle, etc. are displayed on GUI and corresponding parameters such as pressure of IOP, proportion of illumination, degree of vacuum, cutting rate, number of duty cycle, etc. may be adjusted gradually. Each of the control commands is designed as a button or icon on the virtual GUI and the control command and the temporal spatial information of the surgical tool could be associated by location. The user's view changes to Figure 6c when the user starts changing the settings using a surgical tool. In Figure 6c, the surgical tool is placed on a button to decrease the cutting rate of the vitreous cutter. After applying the corresponding control command using the surgical tool as a control input unit, the cutting rate of the vitreous cutter is reduced from 75,000 to 50,000 cpm.

Figures 7a to 7c are schematic diagrams of views of user with different user focuses. The virtual GUI can be displayed in a virtual plane a distance from the surgical site and/or in a periphery of the surgical site. Figure 7a shows the image of the user's view of the surgical site without a virtual GUI and a surgical tool 213. The user's focus is on the surgical site. Figure 7 b shows the image of the user's view of the surgical site with a virtual GUI and a surgical tool 213. The user's focus is on the surgical tool 213 and thus the surgical site is slightly out-of-focus. The control commands/settings of figure 7b are designed as buttons and icons and displayed in the center of the virtual GUI and the control commands/settings could be designed depending on different applications. Figure 7c shows the image of the user's view of the surgical site with a virtual GUI and a surgical tool 213. The user's focus is on the surgical tool 213 and the surgical site is slightly out of focus. The control commands/settings of figure 7c are designed as buttons and icons and displayed in a periphery of the virtual GUI and the control commands/settings could be designed depending on different applications as well.

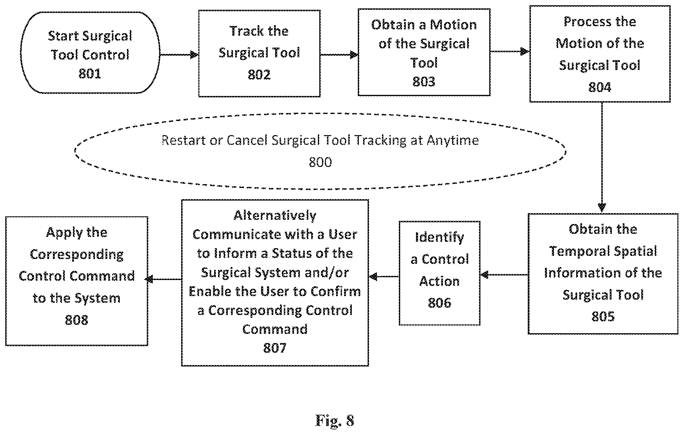

Figure 8 is a representation of one arrangement of the specification of a surgical method outside the scope of the present claims. The method for controlling a surgical system, comprises: starting a surgical tool control mode 801, tracking a surgical tool in a real time 802 to obtain a motion of the surgical tool 803, processing the motion of the surgical tool 804 to obtain a temporal spatial information of the surgical tool 805 (e.g., motion patterns/gestures, location, rotation direction, tool angle, tip proximity from the surgical site, speed, orientation, length, number of repeating, etc.), identifying a control action 806 by associating a control input unit in which control commands are associated with or encoded into various motion patterns/gestures and the temporal spatial information of the surgical tool, alternatively communicate with a user to inform a latest status of the surgical system and/or enable the user to confirm a corresponding control command 807, and applying the corresponding control command to the surgical system 808.

Selectively, restarting or canceling the surgical tool control mode could be performed at any time 800. Reminding or warning the user to move the surgical tool away from a tissue/ the surgical site could be performed (by means of sound, vocal, foot pedal, sensor on the surgical tool, etc.) after starting the surgical tool control mode. Informing the user that applying the corresponding control command is complete could be performed (by means of sound, vocal, foot pedal, sensor on the surgical tool, etc.) after the corresponding control command is applied.

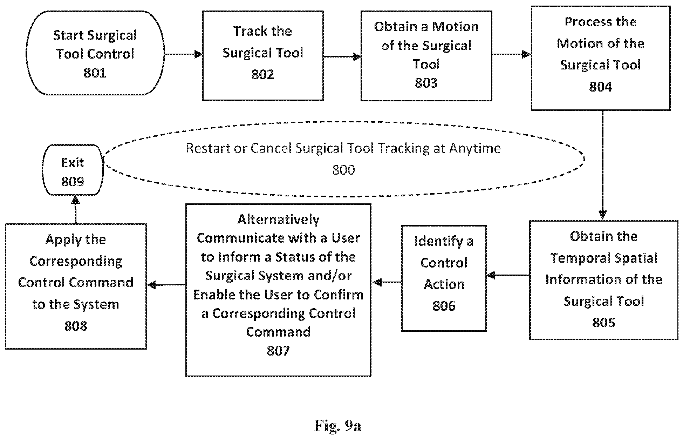

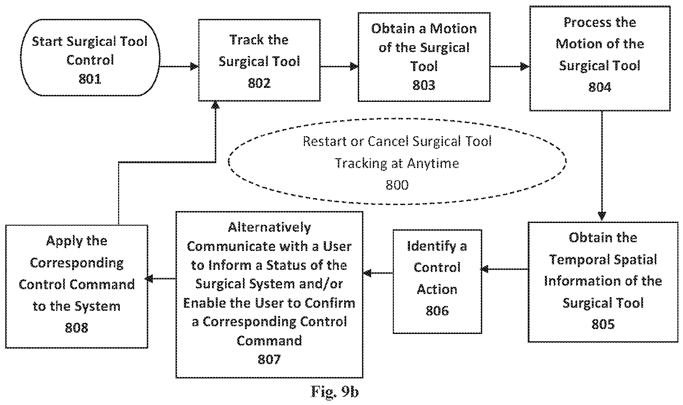

Figure 9a shows a flowchart representing a single control mode for controlling the surgical system. Based on the method of figure 8, the single control mode comprises an additional step: exiting the surgical tool control mode 809 after applying the corresponding control command to the surgical system is performed. Figure 9b shows a flowchart for a continuous control mode of controlling the surgical system. Based on the method of figure 8, the continuous control mode comprises an additional step: re-directing to tracking the surgical tool after applying the corresponding control command to the surgical system.

More specifically, the steps of re-directing to tracking the surgical tool if the control action is not identified or the corresponding control command is not confirmed could be performed any number of times.

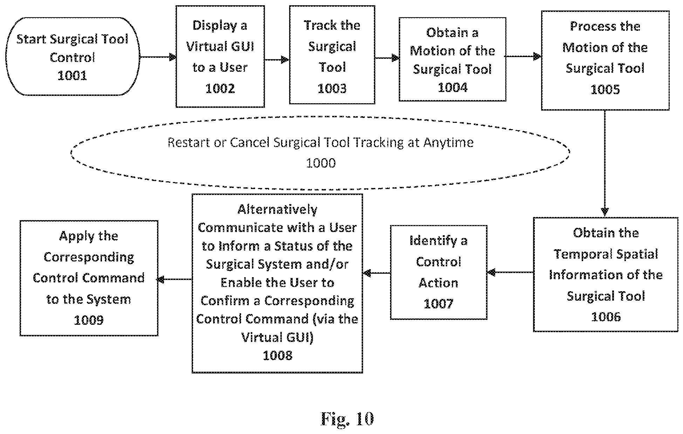

Figure 10 is a representation of one arrangement of the specification of a surgical method outside the scope of the present claims. In the example of Figure 10, a method of using a virtual GUI and a surgical tool as a control input unit for a surgical system is depicted. The method for controlling a surgical system comprises: starting a surgical tool control mode 1001, displaying a virtual GUI to a user 1002, tracking a surgical tool in a real time 1003 to obtain a motion of the surgical tool 1004, processing the motion of the surgical tool 1005 to obtain a temporal spatial information of the surgical tool 1006 (e.g., motion patterns/gestures, location, rotation direction, tool angle, tip proximity from the surgical site, speed, orientation, length, number of repeating, etc.), identifying a control action 1007 by associating a control input unit in which control commands are associated with or encoded into various buttons /icons and the temporal spatial information of the surgical tool, alternatively communicate with the user to inform a latest status of the surgical system and/or enable the user to confirm a corresponding control command (via the virtual GUI) 1008 , and applying the corresponding control command to the surgical system 1009.

Selectively, restarting or canceling the surgical tool control mode could be performed at any time 1000. Reminding or warning the user to move the surgical tool away from a tissue/ the surgical site could be performed (by means of sound, vocal, foot pedal, sensor on the surgical tool, the virtual GUI, etc.) after displaying the virtual GUI. Informing the user that applying the corresponding control command is complete (by means of sound, vocal, foot pedal, sensor on the surgical tool, the virtual GUI, etc.) could be performed after the corresponding control command is applied.

If the control action cannot be identified, tracking the surgical tool will be redirected in order to track the motion of the surgical tool again. If the corresponding control command is not confirmed by the user, the exiting control mode will be directed such that the user could further confirm whether the surgical control mode will be exited. If the user confirms to exit the surgical control mode, the surgical control mode will be ended; if the user confirms not to exit the surgical control mode, the system will start to display the virtual GUI to the user and track the motion of the surgical tool.

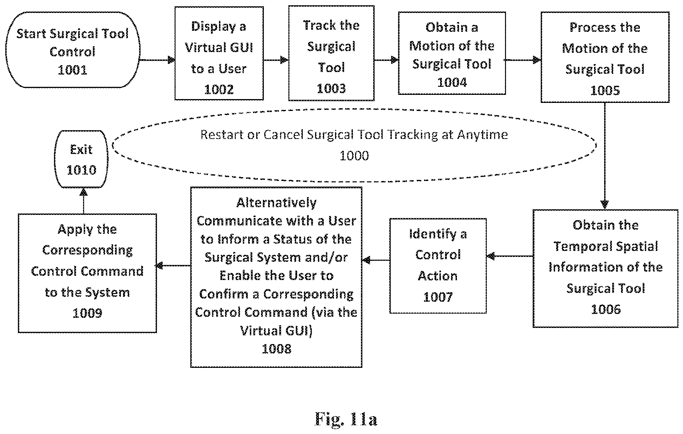

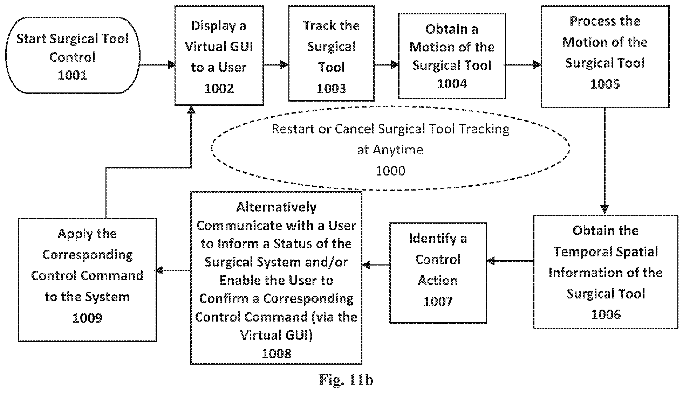

Figure 11a shows a flowchart about a single control mode of controlling the surgical system. The methods are outside the scope of the present claims. Based on the method of figure 10, the single control mode comprises one additional step: exiting the surgical tool control mode 1010 after applying the corresponding control command to the surgical system is performed. Figure 11b shows a flowchart for a continuous control mode of controlling the surgical system. Based on the method of figure 10, the continuous control mode comprises one additional step: re-directing to display the virtual GUI to the user after applying the corresponding control command to the surgical system.

More specifically, the steps of re-directing to tracking the surgical tool if the control action is not identified or the corresponding control command is not confirmed could be performed any number of times.

From the above, it may be appreciated that the present invention provides a surgical system using a surgical tool as a control input so as to empower a surgeon with full control over the surgical settings without increasing the complexity of current surgical consoles.

## Claims

1. A surgical system (100) comprising:
an ophthalmic microscope (211) configured to provide an image of a posterior segment of an eye ;
a control unit (217);
a vitreoretinal surgical tool (213) configured to be placed at a surgical site in the posterior segment of the eye;
a tracking unit configured to track motion of the vitreoretinal surgical tool, while the vitreoretinal surgical tool is located in the posterior segment of the eye, the tracking unit comprising an imaging unit (214) configured to capture more than one image of the vitreoretinal surgical tool and the posterior segment of the eye;
a processing unit (215) for processing the tracked motion of the vitreoretinal surgical tool (213) while the vitreoretinal surgical tool is employed in a surgery in the posterior segment of the eye, wherein the processing unit is configured to receive the captured images and to process and enhance the captured images to extract the motion of the surgical tool so as to obtain temporal spatial information of the tracked motion of a distal end of the vitreoretinal surgical tool;
wherein the control unit (217) further comprises a control input unit (216) comprising a number of control commands, wherein each of the control commands is associated with a motion pattern;
wherein the control unit is configured to identify a control action by associating the control input unit and the temporal spatial information of the vitreoretinal surgical tool to identify whether the temporal spatial information is associated with a motion pattern of a control command of the control input unit and to apply a corresponding control command to the surgical system;
wherein the surgical system further comprises a heads-up display (212) configured in the ophthalmic microscope (211), the heads-up display configured to inform a user of a system status or to enable the user to confirm the corresponding control command.

2. The surgical system of claim 1, wherein the control input unit further comprises a virtual graphic user interface displayed through the heads-up display, and each of the control commands is displayed as an icon in the virtual graphic user interface.

3. The surgical system of claim 2, wherein the virtual graphic user interface is displayed in a virtual plane a distance from the surgical site.

4. The surgical system of claim 3, wherein the control commands are located in a center or periphery of the virtual graphic user interface.

5. The surgical system of claim 1, further comprising:
a second imaging unit (214').

6. The surgical system of claim 5,
wherein the temporal spatial information of the vitreoretinal surgical tool comprises three-dimensional information.

7. The surgical system of claim 1, wherein the tracking unit comprises one or more tracking sensors (222) coupled to the vitreoretinal surgical tool.

## Patentansprüche

1. Chirurgisches System (100), Folgendes umfassend:
ein ophthalmisches Mikroskop (211), das eingerichtet ist, um ein Bild eines hinteren Segments eines Auges bereitzustellen;
eine Steuereinheit (217);
ein vitreoretinales chirurgisches Instrument (213), das eingerichtet ist, um an einem chirurgischen Ort in dem hinteren Segment des Auges angeordnet zu werden;
eine Verfolgungseinheit, die eingerichtet ist, um eine Bewegung des vitreoretinalen chirurgischen Instruments zu verfolgen, während das vitreoretinale chirurgische Instrument in dem hinteren Segment des Auges lokalisiert ist, wobei die Verfolgungseinheit eine Bildgebungseinheit (214) umfasst, die eingerichtet ist, um mehr als ein Bild des vitreoretinalen chirurgischen Instruments und des hinteren Segments des Auges aufzunehmen;
eine Verarbeitungseinheit (215) zum Verarbeiten der verfolgten Bewegung des vitreoretinalen chirurgischen Instruments (213), während das vitreoretinale chirurgische Instrument bei einer Operation in dem hinteren Segment des Auges eingesetzt wird, wobei die Verarbeitungseinheit eingerichtet ist, um die aufgenommenen Bilder zu empfangen und die aufgenommenen Bilder zu verarbeiten und zu verbessern, um die Bewegung des chirurgischen Instruments zu extrahieren, um so Zeit-Raum-Informationen der verfolgten Bewegung eines distalen Endes des vitreoretinalen chirurgischen Instruments zu erlangen;
wobei die Steuereinheit (217) weiterhin eine Steuerungseingabeeinheit (216) umfasst, die eine Anzahl von Steuerungsbefehlen umfasst, wobei jedem der Steuerungsbefehle ein Bewegungsmuster zugeordnet ist;
wobei die Steuereinheit eingerichtet ist, um einen Steuerungsvorgang durch Zuordnen der Steuerungseingabeeinheit und der Zeit-Raum-Informationen des vitreoretinalen chirurgischen Instruments zu identifizieren, um zu identifizieren, ob die Zeit-Raum-Informationen einem Bewegungsmuster eines Steuerungsbefehls der Steuerungseingabeeinheit zugeordnet sind, und um einen entsprechenden Steuerungsbefehl auf das chirurgische System anzuwenden;
wobei das chirurgische System weiterhin eine Informationsanzeige (212) umfasst, die in dem ophthalmischen Mikroskop (211) eingerichtet ist, wobei die Informationsanzeige eingerichtet ist, um einen Benutzer über einen Systemstatus zu informieren oder dem Benutzer zu ermöglichen, den entsprechenden Steuerungsbefehl zu bestätigen.

2. Chirurgisches System nach Anspruch 1, wobei die Steuerungseingabeeinheit weiterhin eine virtuelle grafische Benutzerschnittstelle umfasst, die durch die Informationsanzeige angezeigt wird, und jeder der Steuerungsbefehle als ein Symbol auf der virtuellen grafischen Benutzerschnittstelle angezeigt wird.

3. Chirurgisches System nach Anspruch 2, wobei die virtuelle grafische Benutzerschnittstelle in einer virtuellen Ebene in einem Abstand von dem chirurgischen Ort angezeigt wird.

4. Chirurgisches System nach Anspruch 3, wobei die Steuerungsbefehle in einer Mitte oder an einer Peripherie der virtuellen grafischen Benutzerschnittstelle angeordnet sind.

5. Chirurgisches System nach Anspruch 1, weiterhin Folgendes umfassend:
eine zweite Bildgebungseinheit (214').

6. Chirurgisches System nach Anspruch 5,
wobei die Zeit-Raum-Informationen des vitreoretinalen chirurgischen Instruments dreidimensionale Informationen umfassen.

7. Chirurgisches System nach Anspruch 1, wobei die Verfolgungseinheit mindestens einen Verfolgungssensor (222) umfasst, der mit dem vitreoretinalen chirurgischen Instrument gekoppelt ist.

## Revendications

1. Système chirurgical (100) comprenant :
un microscope ophtalmique (211) configuré pour fournir une image d'un segment postérieur d'un œil ;
une unité de commande (217) ;
un outil chirurgical vitréo-rétinien (213) configuré pour être placé au niveau d'un site chirurgical dans le segment postérieur de l'œil ;
une unité de suivi configurée pour suivre le mouvement de l'outil chirurgical vitréo-rétinien, pendant que l'outil chirurgical vitréo-rétinien se trouve dans le segment postérieur de l'œil, l'unité de suivi comprenant une unité d'imagerie (214) configurée pour capturer plus d'une image de l'outil chirurgical vitréo-rétinien et du segment postérieur de l'œil ;
une unité de traitement (215) pour traiter le mouvement suivi de l'outil chirurgical vitréo-rétinien (213) pendant que l'outil chirurgical vitréo-rétinien est employé dans une intervention chirurgicale dans le segment postérieur de l'œil, dans lequel l'unité de traitement est configurée pour recevoir les images capturées et pour traiter et améliorer les images capturées pour extraire le mouvement de l'outil chirurgical de manière à obtenir des informations spatiotemporelles du mouvement suivi d'une extrémité distale de l'outil chirurgical vitréo-rétinien ;
dans lequel l'unité de commande (217) comprend en outre une unité d'entrée de commande (216) comprenant un certain nombre d'instructions de commande, dans lequel chacune des instructions de commande est associée à un modèle de mouvement ;
dans lequel l'unité de commande est configurée pour identifier une action de commande en associant l'unité d'entrée de commande et les informations spatiotemporelles de l'outil chirurgical vitréo-rétinien pour identifier si les informations spatiotemporelles sont associées à un modèle de mouvement d'une instruction de commande de l'unité d'entrée de commande et pour appliquer une instruction de commande correspondante au système chirurgical ;
dans lequel le système chirurgical comprend en outre un visiocasque (212) configuré dans le microscope ophtalmique (211), le visiocasque étant configuré pour informer un utilisateur d'un état du système ou pour permettre à l'utilisateur de confirmer l'instruction de commande correspondante.

2. Système chirurgical selon la revendication 1, dans lequel l'unité d'entrée de commande comprend en outre une interface graphique utilisateur virtuelle affichée par l'intermédiaire du visiocasque, et chacune des instructions de commande est affichée sous forme d'icône dans l'interface graphique utilisateur virtuelle.

3. Système chirurgical selon la revendication 2, dans lequel l'interface graphique utilisateur virtuelle est affichée dans un plan virtuel à une certaine distance du site chirurgical.

4. Système chirurgical selon la revendication 3, dans lequel les instructions de commande sont situées dans un centre ou une périphérie de l'interface graphique utilisateur virtuelle.

5. Système chirurgical selon la revendication 1, comprenant en outre :
une seconde unité d'imagerie (214').

6. Système chirurgical selon la revendication 5, dans lequel les informations spatiotemporelles de l'outil chirurgical vitréo-rétinien comprennent des informations tridimensionnelles.

7. Système chirurgical selon la revendication 1, dans lequel l'unité de suivi comprend un ou plusieurs capteurs de suivi (222) couplés à l'outil chirurgical vitréo-rétinien.
